(19) Europäisches Patentamt
European Patent Office
Office européen des brevets

(11) **EP 3 377 682 B1**

(12) **EUROPEAN PATENT SPECIFICATION**

(45) Date of publication and mention
of the grant of the patent:
**31.03.2021 Bulletin 2021/13**

(51) Int Cl.:
***D01D 5/06*** *(2006.01)*      ***D01F 2/28*** *(2006.01)*
***D01F 9/04*** *(2006.01)*

(21) Application number: **15801217.9**

(86) International application number:
**PCT/GB2015/053509**

(22) Date of filing: **18.11.2015**

(87) International publication number:
**WO 2017/085436 (26.05.2017 Gazette 2017/21)**

(54) **GELLING FIBRES**

GELIERENDE FASERN

FIBRES DE GÉLIFICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.09.2018 Bulletin 2018/39**

(73) Proprietor: **Advanced Medical Solutions Limited Winsford, Cheshire CW7 3RT (GB)**

(72) Inventors:
• **BRADFORD, Colin Raymond
Keighley
West Yorkshire BD20 9AZ (GB)**
• **HORRIDGE, David
Sandbach
Cheshire CW11 3BP (GB)**

(74) Representative: **Stafford, Jonathan Alan Lewis
Marks & Clerk LLP
1 New York Street
Manchester M1 4HD (GB)**

(56) References cited:
**WO-A1-02/36866**     **CN-A- 103 060 946**
**US-A- 6 140 257**

• **MARI PANTSAR-KALLIO ET AL: "Determination of sodium, potassium, calcium and magnesium cations by capillary electrophoresis compared with ion chromatography", ANALYTICA CHIMICA ACTA, vol. 314, no. 1-2, 10 October 1995 (1995-10-10), pages 67-75, XP055247358, DOI: 10.1016/0003-2670(95)00266-3**
• **M. W. WELCH ET AL: "Method Comparison for Calcium Determination by Flame Atomic Absorption Spectrophotometry in the Presence of Phosphate", CLINICAL CHEMISTRY, vol. 36, no. 2, 1 February 1990 (1990-02-01), pages 351-354, XP055247378,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to fibres which are useful particularly, but not necessarily exclusively, in the manufacture of wound dressings. More particularly the invention relates to fibres which have high absorbency for an aqueous fluid (e.g. wound exudate) and which are capable of forming a gel upon absorption of sufficient aqueous fluid. Fibres possessing this characteristic are known in the art as "gelling fibres".

The use of gelling fibres in the manufacture of wound dressings is well established. The dressing may, for example, be in the form of a non-woven felt. When wound dressings made from gelling fibres are applied to a wound (such application often being in conjunction with a secondary dressing such as a breathable film that overlies the fibrous dressing) exudate from the wound is absorbed by the dressing which results in the dressing converting it into the form of a gel. Such gels are advantageous in wound care because they maintain an ideal moist wound environment and "lock-in" the wound exudate that has been absorbed. The fibres used for producing the wound dressing generally produce a gel of sufficient integrity as not to disintegrate within the wound, at least within the time for which the dressing is intended to remain in place, so the dressing may be removed intact from the wound.

One type of gelling fibre used in the manufacture of wound dressings is carboxymethyl cellulose (CMC). Such CMC fibres for use in wound dressings are prepared by carboxymethylation of pre-prepared cellulose fibres (cellulose fibres are also known as viscose or rayon). This approach (i.e. carboxymethylation of a pre-prepared fibre) is adopted because CMC in powder form is not capable of forming fibres by conventional processes such as spinning. Thus it is necessary to pre-prepare fibres (of a fibre-forming material such as cellulose) that can be carboxymethylated. Thus, for example, EP 0 616 650 discloses a process in which solvent-spun cellulose fibre is carboxymethylated by reaction with a solution containing a strong alkali (e.g. sodium hydroxide) and a monochloroacetate reagent (e.g. sodium chloroacetate). The treated fibres are dried then washed with a solution containing 50% industrial ethanol. The degree of substitution is controlled by the concentration of the components in the reaction solution and the processing conditions.

The process summarised in the previous paragraph requires the use of strong alkalis, chemicals harmful to personnel and the environment, and also flammable solvents. The process therefore needs to be conducted in a controlled environment with EX-rated equipment that is safe with flammable and explosive vapours.

The inability of CMC powder *per se* to be fibre forming is in contrast to alginates which readily form fibres by conventional fibre forming techniques, such as by spinning an aqueous solution ("dope") of a soluble form of the alginate (e.g. the sodium salt) through a spinneret into a coagulation bath containing multi-valent cations (usually calcium) that cross-link the alginate to form fibres.

Alginate fibres produced in this way are highly absorbent and are extensively used in the manufacture of wound dressings. It is known that alginate fibres of improved absorption (as compared to fibres comprising alginate alone) may be produced by co-spinning a solution which comprises dissolved alginate and an absorption-enhancing organic polymeric species into a coagulation bath. In this regard, US 6,080,420 is concerned with improving the absorbency of alginate fibres and discloses the production of alginate fibres by the spinning of an aqueous solution containing dissolved alginate (usually sodium alginate) and at least one dissolved absorption-enhancing organic polymeric species. US 6,080,420 discloses that the fibres formed generally comprise a major proportion by weight of the alginate, e.g. 50-95% by weight, and a minor proportion of the absorption-enhancing polymeric species. A range of such organic polymeric species is disclosed including uncharged and polyanionic species, e.g. polymers with $COO^-$ or $SO_4^{2-}$ groups along the polymer chain. More particularly, US 6,080,420 proposes that fibres may be co-spun with a polysaccharide (other than an alginate) containing $COO^-$ groups, examples of such polysaccharides being carboxymethyl cellulose and carrageenan. Fibres of improved absorbency may comprise 70-90% of the alginate and a total of 5-30% of the aforementioned polysaccharides. Example 2 of US 6,080,420 discloses production of a fibre from a solution containing 13.5 kg of sodium alginate and 1.5 kg of sodium carboxymethyl cellulose (producing a fibre containing about 10% by weight (CMC). Fibres comprising carboxymethyl cellulose and alginate are described also in US6,140,257.

According to a first aspect of the present invention there is provided fibres comprising carboxymethyl cellulose and an alginate in a total amount of at least 90% by weight of the fibre, the carboxymethyl cellulose and alginate being distributed homogeneously throughout the fibre, wherein:

   (a) the amount of carboxymethyl cellulose present in the fibre is at least 50% by weight of the total weight of the carboxymethyl cellulose and alginate;
   (b) the alginate has a Guluronate-content of 50-90% by weight, and
   (c) the value of z given by the expression

$$z = \frac{(2x/40)}{(2x/40) + (y/23)} \qquad (1)$$

where x and y are the percentages by weight of calcium and sodium respectively in the fibres is at least 80%.

According to a second aspect of the present invention there is provided a method of producing fibres comprising spinning a dope which is an aqueous solution containing dissolved carboxymethyl cellulose and dissolved alginate into a coagulation bath containing dissolved calcium ions that cause cross-linking of the alginate and precipitation of fibres comprised of the alginate and polysaccharide characterized in that:

(a) the amount of carboxymethyl cellulose in the dope is at least 50% by weight of the total weight of the carboxymethyl cellulose and alginate,
(b) the sodium alginate has a Guluronate-content of 50-90% by weight,
(c) the concentration of calcium ions in the coagulation bath provides fibres in which the value of z in the expression

$$z = \frac{(2x/40)}{(2x/40) + (y/23)} \qquad (1)$$

where x and y are the percentages by weight of calcium and sodium respectively in the fibres is at least 80%, and
(d) the fibres produced contain carboxymethyl cellulose and alginate in a total amount of at least 90% by weight of the fibre.

As indicated above, the gelling characteristics of the fibres of the invention are achieved by the presence in the fibre of at least 50% by weight (and preferably more than 50% by weight) of CMC based on the total weight of alginate and CMC (the fibre itself comprising at least 90% by weight of the combined amount of alginate and CMC). The ability to achieve such high loadings of CMC (which is not *per se* fibre forming) is achieved by observing certain characteristics for the fibres. The first of these characteristics is that the alginate in the fibre is one having a Guluronate ("G") content of 50-90% by weight and correspondingly a Manuronate ("M") content of 10-50% by weight. The second characteristic is that the value of z as given by the expression

$$z = \frac{(2x/40)}{(2x/40) + (y/23)} \qquad (1)$$

where x and y are the percentages by weight of calcium and sodium respectively in the fibres is at least 80%.
With regard to the first of these characteristics, we have found that alginates with an M-content of greater than 50% are not able to produce fibres comprising at least 50% by weight of CMC based on the combined total weight of CMC and alginate (which together provide at least 90% by weight of the fibre). Alginates with a G-content of 50-90% by weight generally come from the stems of seaweed and produce higher wet strength fibres but a lower level of gelling. Alginates with an M-content of greater than 50% by weight generally come from the leaves of seaweed and produce fibres with a higher level of gelling but a lower wet strength. Typically the alginate such that a 1% by weight solution will have a viscosity of 10-300 cP, preferably 20-80 cP.
With regard to the second of the two characteristics above, the value of z is a representation of the total amount of positive charge provided by the calcium ions (taking into account that calcium is a 2+ ion and has an atomic weight of 40) relative to the total amount of charge provided by the calcium and sodium ions (taking into account the fact that the latter is a 1+ ion and has an atomic weight of 23). The value of z therefore is representative of the extent to which calcium ions (from the coagulation bath) have "displaced" sodium ions in the original sodium alginate to effect cross-linking of the fibre. With a value of z of at least 80, we have found that there is sufficient cross-linking of the alginate to provide a matrix capable of supporting the relatively high amount of "non-fibre forming" CMC in the fibre. Preferably the value of z is at least 85 and more preferably at least 90. Preferably the value of "x" (i.e. the percent by weight of calcium in the fibre) is 5.8 to 8.1% by weight. Preferably also the value of "y" (i.e. the weight percent of sodium in the fibres) is .04 to 03% by weight. The values of "x" and "y" are determined by conventional analytical techniques, as atomic absorption spectroscopy.
The present invention is in contrast to the disclosure of US 6,080,420. As indicated above, the disclosure of that patent is directed to improving the absorbency of alginate fibres and does so by the incorporation therein (in certain embodiments) of a minor amount of carboxymethyl cellulose (or other polysaccharide). In contrast, and very surprisingly, we have now found that it is possible to produce fibres which comprise a major amount (i.e. more than 50% by weight) of CMC and correspondingly a minor amount of alginate. The underlying rationale of the fibres of the present invention is different from those produced in US 6,080,420. The latter is concerned with the production of alginate fibres and modifying the absorbency thereof. In contrast, the present invention provides a fibre that is comprised predominantly of CMC for the purposes of obtaining the benefit of the absorption and gelling characteristics thereof, the alginate being present in the fibre as a support matrix for the high CMC loading (albeit it that the alginate will itself contribute to overall absorbency of the fibre).

Fibres in accordance with the invention are gelling fibres and have excellent absorption characteristics. As such, the fibres are eminently suitable for use in the manufacture of wound dressings. Such dressings, may for example, be in the form of non-woven felts produced from staple fibres by standard techniques of fibre opening, carding, cross-lapping and needling.

The invention provides the additional advantage that the fibres may be produced by spinning a solution containing dissolved CMC and dissolved sodium alginate into a coagulation bath so that the invention avoids the need for the carboxymethylation of pre-formed fibres with the attendant chemical processes involved therein.

Generally, fibres in accordance with the invention will comprise at least 55% by weight of CMC and at most 45% by weight of alginate (both percentages being based on the total dry weight of the CMC and alginate in the fibre). More preferred fibres comprise at least 60% by weight of CMC and at most 40% by weight of alginate on the same percentage basis.

The fibres comprise a total of at least 90%, preferably at least 95%, by dry weight of the CMC and alginate. The fibres may, in total, comprise 100% by weight of the CMC and alginate. If the total amount of the CMC and alginate in the fibres is less than 100% by weight (but more than the required minimum of 90% by weight) the balance may be components imparting desirable end properties for the fibres. Where the fibres are intended for use in wound dressings, they may for example incorporate an antimicrobial agent such as silver, or other actives that may be beneficial to wound healing

The fibres may be produced by spinning through a spinneret, of a solution containing dissolved carboxymethyl cellulose (e.g. the sodium salt thereof) and dissolved sodium alginate into a coagulation bath containing a solution of calcium cations to produce fibres by virtue of the calcium ions displacing sodium ions from the alginate to effect cross-linking thereof and produce a solid fibre incorporating the CMC. The solution to be spun will comprise dissolved CMC and alginate in the proportions required in the final fibre. For a given spinning dope (comprising pre-defined concentrations of a particular alginate and a particular CMC) the concentration of calcium ions in the coagulation bath to produce fibres for which the value of "z" (equation (1) above) is at least 80 can readily be determined. Analysis of the fibres (by using atomic absorption spectroscopy) to determine the percentage by weights of sodium and calcium in the fibres gives the values of "x" and "y" required, in equation (1), for the calculation of "z". If "z" does not have the required value then the concentration of calcium in the coagulation bath may be readily adjusted (e.g. in incremental steps) until it is found that, on analysis, the resulting fibres have values of "x" and "y" but give the required value for "z".

Typically, the solution will comprise at least 3% by weight of total amount of dissolved CMC and alginate, although generally not more than 8% on the same basis. More usually, the solution to be spun will comprise 5-6% on the same basis. A preferred calcium salt for use in formulating the coagulation bath is calcium chloride, e.g. used in the dihydrate form ($CaCl_2.2H_2O$). The coagulation bath will preferably be a 2%-3% w/w (e.g. about 2.4% w/w) solution of calcium chloride dihydrate. Other calcium salts may be used in amounts to give similar calcium concentrations. Thus, at the more general level, the coagulation bath will have a calcium concentration in the range of 0.13 to 0.21 moles per litre (preferably about 0.16 moles per litre).

The carboxymethyl cellulose used for the process will preferably be such that it has a degree of substitution of at least 0.65, a viscosity of a 2% solution of 200-800cP and a purity of at least 99%.

The dope may also contain any additional component required for incorporation in the fibre, e.g. an antimicrobial agent or active.

Fibres in accordance with the invention manufactured by an in-line process in which fibres are produced by spinning an aqueous solution ("dope") containing dissolved CMC and alginate into a coagulation bath to produce fibres may be collected into a tow and then subjected to downstream processing steps to produce fibres in a suitable form for conversion into a wound dressing. These downstream processing steps generally include orientation, washing (to remove surface salts), water removal by passage through a series of baths containing increasing concentrations of acetone, mechanical removal (e.g. by means of a mangle) to remove the bulk of the remaining acetone and water, and drying in an oven. A spin finish may be applied. The tow is crimped and then cut into staple fibres.

Fibres in accordance with the invention may be produced by an in-line process of the same general type as that described above for the production of alginate fibres containing a minor (e.g. 15%) proportion by weight of CMC. The fibres as formed in the coagulation bath are however inherently weaker than alginate fibres at the same stage (whether or not the alginate fibres also contain a minor proportion of CMC) due to their higher loading of CMC (a polymer that is not fibre forming). To prevent fibre breakage, care must be taken to ensure that the fibres are created and processed in a way that individual fibres are initially formed, not subjected to too much tension in the upstream stages of the process, and have their strength enhanced as they progress through the process to produce a final product suitable for conversion into a product such as a wound dressing.

An in-line process as outlined below is suitable for producing the fibres.

Preparation of the dope is an important first step in the process. The dope is prepared by dissolution of a water soluble carboxymethyl cellulose (usually sodium CMC) and sodium alginate. Generally the total amount of the CMC salt and alginate salt in the dope will be a minimum of 3% but generally not more than 8%. More generally, the total amount of the CMC salt and alginate salt will be in the range 5-6%. It is important that the solution is mixed thoroughly and to all intents-and-purposes there are no undissolved salts in the dope. This is best achieved by use of a mixing system in which powders are added by vacuum into a recirculating stream of water via a homogenizer. The production of a homogeneous solution at this stage is important because it ensures that the CMC salt and alginate salt form a solution in which the weight ratio of CMC salt to alginate salt is the same as in the dry powder mix and therefore represents the intended ratio in the fibres. If mixing is less efficient, e.g. by using a propeller stirrer or similar, a proportion (not necessarily the same) of each of the CMC salt and alginate salt remain undissolved, generally in the form of a "jelly", and excessive insoluble material needs to be filtered before spinning of the solution through the spinneret (with its small cross-section apertures) can be begin. Not only does this require frequent cleaning/replacement of a filter upstream of the spinneret, but also the ratio of dissolved CMC salt to dissolved alginate salt will be different from that of the dry powder mix.

It is essential that the dope is degassed, prior to spinning. Air bubbles in the dope will cause fibre breakages, and weaken the whole tow, possibly to a level where it will not survive the remaining downstream processes. The addition of powders under vacuum reduces the potential for air bubbles.

We have found that an important feature in the overall process for successful production of high CMC/alginate fibres is the generation of individual fibres of adequate strength to survive the process to the point where they are formed into a tow, and subsequent further processing of the tow that maintains the integrity of both the tow and the individual fibres.

Generation of fibres of adequate strength in the spinning bath is an important feature of the upstream end of the process. The fibres are produced by pumping the dope through a spinneret into an aqueous solution containing calcium ions which serve to cross-link the alginate and form a fibrous structure in which the CMC is entrapped within a matrix formed by the alginate. Important to this process is the concentration of the calcium ions in the coagulation bath. For the successful production of high CMC/alginate fibres we have found it necessary for the concentration of the calcium ions in the coagulation bath to be somewhat higher than that typically used for the production of prior art alginate/CMC fibres containing a minor proportion of CMC. Coagulation baths used in the production of such prior art fibres typically contain about 1.5% by weight calcium chloride dihydrate (expressed as $CaCl_2.2H_2O$). However, attempts to produced high CMC/alginate fibres using this level (1.5% by weight) of calcium chloride have been found to yield high CMC/alginate fibres that do not have adequate strength to survive the production process. Two adaptations have been found to be necessary. The first is to use a higher concentration of calcium ions, typically provided by at least 2% by weight calcium chloride dihydrate (expressed as $CaCl_2.H_2O$) and accurately to maintain the calcium level in the bath. This requires monitoring of the calcium level in the solution and adding calcium chloride as necessary to maintain the desired higher calcium ion concentration.

On exit from the coagulation bath, the freshly spun fibres converge to form a tow which is subjected to downstream processing operations as described more fully below. Strength of the tow at this point (i.e. where it exits the coagulation bath) has been found to be an important feature of the production process since, if too weak, the tow simply breaks. Forming the fibres as described in the previous paragraph (i.e. with the use of a maintained high calcium concentration) assists in this regard but may not of itself be sufficient. The required strength of the tow may be achieved by simultaneously spinning (into the coagulation bath) a large number of fibres (e.g. >75,000) which, when combined, provide a tow of adequate strength.

Formation of a tow of adequate strength allows the fibres to be transferred from the coagulation bath to the next downstream processing stage where conditions must be such as to maintain the integrity of both the tow and the individual fibres. Each of the downstream stages described more fully below are operated under conditions that meet this requirement.

The tow formed from the fibres leaving the coagulation bath is subjected to a stretching operation in water at a temperature of greater than 35°C. Stretching is achieved by passing the tow from the coagulation bath in a serpentine path around a first set of rollers, through the hot water bath and then again in a serpentine path around a second set of rollers running faster than the first set. The stretch ratio, i.e. the speed of the second set of rollers divided by the speed of the first set, is at least 125%, but not sufficient that it causes fibre breakages, and is generally less than 200% Stretching of the tow in this way orients the polymer chains in the fibres and imparts strength thereto.

The stretched fibres are then passed through a series of cold water baths to remove any residual salt from the fibres. It also prevents residual CMC that may have come out of the tow transferring to downstream processes.

After washing, the fibres need to be subjected to a water removal step and subsequent drying. Even at the water removal stage, the fibres are still relatively fragile (although the tow is still intact because of the precautions taken upstream) and needs to be subjected to a water removal step that is relatively gentle. This can be achieved by passing the tow successively through aqueous acetone baths containing increasing concentrations of acetone, typically from 35% to 95% acetone. This gentle water removal procedure reduces breakages and makes the fibres easier to process.

After passage through the water/acetone baths, excess liquid is mechanically removed from the fibres, e.g. by passage through the nip of at least one pair of rollers.

Final drying of the tow may then be effected by passing the tow through an oven or other drying system, but leaving some moisture in the fibres (typically 5-15%).

The fibres thus formed are stable and can be subjected to conventional fibre processing conditions, e.g. application of a spin finish (to assist subsequent carding), crimping and cutting into stable fibres. Such fibres can then be converted into wound dressings by the steps of:

(a) fibre opening - a coarse combing action to separate fibre clumps into individual fibres;
(b) carding - a fine combing operation to separate all fibres and create a coherent lightweight web, typically having a weight of 10-20 g/m$^2$;
(c) cross-lapping - to lay down overlapping layers of the lightweight, carded web to build up the overall weight to the required level, e.g. 100-300 g/m$^2$; and
(d) needling in which a plurality of barbed needles penetrate the fibres, taking fibres from the surface of the web into the middle and thereby entangling the fibres to create a felt.

Alternatively, the staple fibres can be spun into yarns, similar to natural fibres such as cotton. Woven or knitted fabrics can be produced from the yarns. However, by their nature, yarns restrict the gelling of the fibres, and hence reduce absorbency, but are strong and given an integral product.

A combination of the two technologies would be possible by needling fibres into a woven or knitted fabric, which would give strength and absorbency.

Also it will be possible to needle fibres into substrates manufactured from other materials such as spun laid non-wovens, woven substrates or knitted materials.

The invention is illustrated with reference to the following non-limiting Example and the accompanying drawing, in which:

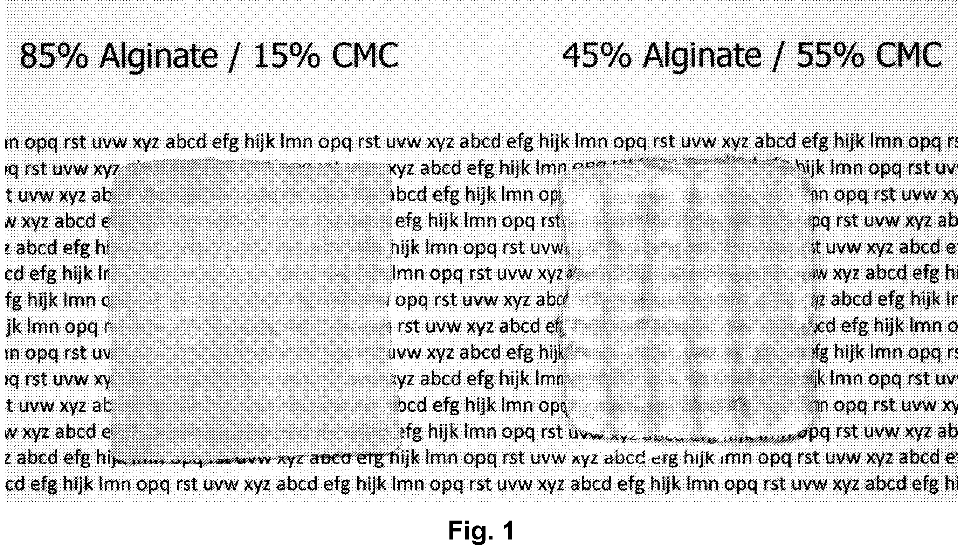

Fig. 1 illustrates wetted samples of felts produced from (a) fibres in accordance with the invention, and (b) comparative fibres, the felts being shown against a background of random text to demonstrate their relative translucency.

**Example**

This Example describes preparation of fibres in accordance with the invention and utilises a procedure as described more fully above.

55kg of sodium carboxymethyl cellulose powder was dissolved in 1718 litres of pure water using a mixing system in which the CMC powder was added by vacuum into a recirculating stream of water via a homogenizer. Once the CMC had been fully dissolved, 44kg of a High G sodium alginate powder (G content 65-70%) was added and dissolved (using the same mixing system) to produce a homogenous solution ("dope").

The dope was allowed to degas for 30 hours.

The dope was pumped through spinnerets producing a total of 80,000 filaments, into a coagulation bath containing dissolved calcium chloride dihydrate maintained in an amount of 2-3% w.w (expressed as CaCl$_2$.2H$_2$O).

The fibres produced in the coagulation bath were collected to form a tow which was subjected to a stretching operation in water at a temperature of 50-60°C, the stretch ratio being 150-160%.

After stretching, the fibres were passed through a series of cold water baths to remove residual salt. The washed fibres were then passed successively through acetone baths containing increasing concentrations of acetone (35% to 95%).

Excess liquid was then mechanically removed from the fibres by passage through the nip between a pair of rollers. The tow was then passed through an oven to reduce residual moisture content to 5-15% by weight.

A spin finish of Polysorbate 20 was then applied to the fibres at a level of 0.5-2.0%, prior to the fibres being crimped and then cut into 50mm staple lengths.

A non-woven felt having a weight of about 220 g m$^{-2}$ was produced from the staple fibres.

For the purposes of comparison, the above spinning procedure was repeated but to produce fibres comprising 15% by weight of the CMC and 85% by weight of the alginate. Again using the procedure outlined above, these fibres were used to produce a felt having a weight of about 220 g m$^{-2}$.

Analysis of both fibre types demonstrated that (prior to application of the spin finish) the fibres had a value of "z" as determined by equation (1) in excess of 90.

Samples of the two felts (area = 120 cm$^{-2}$) were saturated with Solution A (8.298g of sodium chloride and 0.368g of calcium chloride made up to 1 litre with purified water) and allowed to drain.

The felt produced from the fibres comprising 55% CMC and 45% alginate had a higher absorption than the felt produced from the fibres comprising 85% alginate and 15% CMC. Additionally, the fibres in the felt produced from the fibres comprising 55% CMC and 45% alginate were gelled and the wetted felt had a translucent appearance. In contrast, the fibres comprising 85% alginate and 15% CMC did not gel and the felt produced therefrom remained generally opaque.

The test results as summarised in the previous paragraph are shown in Fig. 1 of the accompanying drawings in which the wetted felts are shown against a background of random text. The translucent nature of the felt produced form the fibres comprising 55% CMC and 45% alginate can clearly be seen.

**Claims**

1. Fibres comprising carboxymethyl cellulose and alginate in a total amount of at least 90% by weight of the fibre, the carboxymethyl cellulose and alginate being homogeneously distributed throughout the fibre, wherein:

   (a) the amount of carboxymethyl cellulose present in the fibre is at least 50% by weight of the total weight of the carboxymethyl cellulose and alginate;
   (b) the alginate has a Guluronate-content of 50-90% by weight, and
   (c) the value of z given by the expression

$$z = \frac{(2x/40)}{(2x/40) + (y/23)} \qquad (1)$$

   where x and y are the percentages by weight of calcium and sodium respectively in the fibres is at least 80 %, as determined by atomic absorption spectroscopy.

2. Fibres as claimed in claim 1 wherein the total amount of carboxymethyl cellulose and alginate present in the fibre is at least 95% by weight of the fibre.

3. Fibres as claimed in claim 1 comprising at least 55% by weight of the carboxymethyl cellulose and at most 45% by weight of the alginate based on the total weight of the alginate and carboxymethyl cellulose.

4. Fibres as claimed in claim 1 to 3 wherein the carboxymethyl cellulose has a degree of carboxymethylation of at least 0.65.

5. Fibres as claimed in claim 1 wherein the alginate has a Guluronate-content of 50-80% by weight and an M-content of 20-50% by weight.

6. Fibres as claimed in any one of claims 1 to 5 wherein the value of z is at least 85 %; optionally wherein the value of z is at least 90 %.

7. Fibres as claimed in any one of claims 1 to 6 produced by co-spinning an aqueous dope comprising dissolved carboxymethyl cellulose and dissolved alginate into a coagulation bath.

8. Fibres as claimed in any one of claims 1 to 7 in staple form.

9. A non-woven material which comprises entangled fibres as claimed in any one of the preceding claims.

10. A non-woven material as claimed in claim 9 in the form of a non-woven needled felt; optionally in combination with a substrate into which the fibres of the material are needled.

11. A woven material comprising fibres as claimed in any one of claims 1 to 8.

12. A knitted material comprising fibres as claimed in any one of claims 1 to 8.

13. A wound dressing comprising a material as claimed in any one of claims 9 to 12; optionally wherein said material forms one layer of a multi-layer dressing.

14. A method of producing fibres comprising spinning a dope which is an aqueous solution containing dissolved carboxymethyl cellulose and dissolved alginate into a coagulation bath containing dissolved calcium ions that cause cross-linking of the alginate and precipitation of fibres comprised of the alginate and polysaccharide **characterized in that**:

(a) the amount of carboxymethyl cellulose in the dope is at least 50% by weight of the total weight of the carboxymethyl cellulose and alginate,
(b) the sodium alginate has a Guluronate-content of 50-90% by weight,
(c) the concentration of calcium ions in the coagulation bath provides fibres in which the value of z in the expression

$$z = \frac{(2x/40)}{(2x/40) + (y/23)} \qquad (1)$$

where x and y are the percentages by weight of calcium and sodium respectively in the fibres is at least 80 % as determined by atomic absorption spectroscopy, and
(d) the fibres produced contain carboxymethyl cellulose and alginate in a total amount of at least 90% by weight of the fibre.

15. A method as claimed in claim 14 wherein the amount of carboxymethyl cellulose in the dope is at least 55% by weight of the total weight of the carboxymethyl cellulose and alginate; optionally wherein the amount of carboxymethyl cellulose in the dope is at least 60% by weight of the total weight of the carboxymethyl cellulose and alginate.

**Patentansprüche**

1. Fasern, umfassend Carboxymethylzellulose und Alginat in einer Gesamtmenge von mindestens 90 Gewichtsprozent der Fasern, wobei die Carboxymethylzellulose und das Alginat homogen durch die Fasern verteilt sind, wobei:

(a) die Menge an in den Fasern vorhandener Carboxymethylzellulose mindestens 50 Gewichtsprozent des Gesamtgewichts von Carboxymethylzellulose und Alginat beträgt;
(b) das Alginat einen Guluronatgehalt von 50-90 % Gewichtsprozent aufweist, und
(c) der Wert von z, welcher sich aus folgendem Ausdruck ergibt:

$$z = \frac{(2x/40)}{(2x/40) + (y/23)} \qquad (1)$$

wobei x und y die jeweiligen Gewichtsprozentsätze von Kalzium und Natrium in den Fasern sind, mindestens 80 % beträgt, wie durch Atomabsorptionsspektroskopie bestimmt.

2. Fasern nach Anspruch 1, wobei die Gesamtmenge an in den Fasern vorhandener Carboxymethylzellulose und darin vorhandenem Alginat mindestens 95 Gewichtsprozent der Fasern beträgt.

3. Fasern nach Anspruch 1, umfassend mindestens 55 Gewichtsprozent der Carboxymethylzellulose und maximal 45 Gewichtsprozent des Alginats, basierend auf dem Gesamtgewicht des Alginats und der Carboxymethylzellulose.

4. Fasern nach Anspruch 1 bis 3, wobei die Carboxymethylzellulose einen Grad der Carboxymethylierung von mindestens 0,65 aufweist.

5. Fasern nach Anspruch 1, wobei das Alginat einen Guluronatgehalt von 50-80 Gewichtsprozent und einen M-Gehalt von 20-50 Gewichtsprozent aufweist.

6. Fasern nach einem der Ansprüche 1 bis 5, wobei der Wert von z mindestens 85 % beträgt; wobei optionsweise der Wert von z mindestens 90 % beträgt.

7. Fasern nach einem der Ansprüche 1 bis 6, erzeugt durch Co-Spinnen einer wässrigen Dotierungslösung, welche aufgelöste Carboxymethylzellulose und aufgelöstes Alginat umfasst, in ein Koagulationsbad.

8. Fasern nach einem der Ansprüche 1 bis 7 in Klammerform.

9. Vliesmaterial, welches verschlungene Fasern nach einem der vorhergehenden Ansprüche umfasst.

10. Vliesmaterial nach Anspruch 9 in der Form eines Nadelvlies-Filzes; optionsweise in Kombination mit einem Substrat, in welches die Fasern des Materials genadelt werden.

11. Vliesmaterial, umfassend Fasern nach einem der Ansprüche 1 bis 8.

12. Strickmaterial, umfassend Fasern nach einem der Ansprüche 1 bis 8.

13. Wundverband, umfassend ein Material nach einem der Ansprüche 9 bis 12; wobei optionsweise das Material eine Schicht eines mehrlagigen Verbandes bildet.

14. Verfahren zum Erzeugen von Fasern, umfassend Spinnen einer Dotierungslösung, welche eine wässrige Lösung ist, welche aufgelöste Carboxymethylzellulose und aufgelöstes Alginat umfasst, in einem Koagulationsbad, welches aufgelöste Kalziumionen enthält, welche eine Vernetzung des Alginats und eine Ausfällung von Fasern verursachen, welche aus dem Alginat und aus Polysaccharid bestehen, **dadurch gekennzeichnet, dass**:

(a) die Menge an in den Fasern vorhandener Dotierungslösung mindestens 50 Gewichtsprozent des Gesamtgewichts von Carboxymethylzellulose und Alginat beträgt;
(b) das Natriumalginat einen Guluronatgehalt von 50-90 % Gewichtsprozent aufweist,
(c) die Konzentration an Kalziumionen in dem Koagulationsbad Fasern bereitstellt, in welchen der Wert von z, sich aus folgendem Ausdruck ergibt:

$$z = \frac{(2x/40)}{(2x/40) + (y/23)} \qquad (1)$$

wobei x und y die jeweiligen Gewichtsprozentsätze von Kalzium und Natrium in den Fasern sind, mindestens 80 % beträgt, wie durch Atomabsorptionsspektroskopie bestimmt.
(d) die erzeugten Fasern Carboxymethylzellulose und Alginat ein einer Gesamtmenge von mindestens 90 Gewichtsprozent der Fasern enthalten.

15. Verfahren nach Anspruch 14, wobei die Menge an Carboxymethylzellulose in der Dotierungslösung mindestens 55 Gewichtsprozent des Gesamtgewichts der Carboxymethylzellulose und des Alginats beträgt; wobei optionsweise die Menge an Carboxymethylzellulose in der Dotierungslösung mindestens 60 Gewichtsprozent des Gesamtgewichts der Carboxymethylzellulose und des Alginats beträgt.

**Revendications**

1. Fibres comprenant une carboxyméthylcellulose et un alginate dans une quantité totale d'au moins 90% en poids de la fibre, la carboxyméthylcellulose et l'alginate étant distribués de façon homogène dans toute la fibre, dans lesquelles:

(a) la quantité de carboxyméthylcellulose présente dans la fibre est d'au moins 50% en poids du poids total de la carboxyméthylcellulose et de l'alginate,
(b) l'alginate a une teneur en guluronate de 50-90% en poids, et
(c) la valeur de z donnée par l'expression:

$$z = \frac{(2x/40)}{(2x/40)+(y/23)} \qquad (1)$$

où x et y sont les pourcentages en poids de calcium et de sodium respectivement dans les fibres, est d'au moins 80%, telle que déterminée par une spectroscopie d'absorption atomique.

2. Fibres selon la revendication 1, dans laquelle la quantité totale de carboxyméthylcellulose et d'alginate présents dans la fibre est d'au moins 95% en poids de la fibre.

3. Fibres selon la revendication 1, comprenant au moins 55% en poids de la carboxyméthylcellulose et au plus 45% en poids de l'alginate sur la base du poids total de l'alginate et de la carboxyméthylcellulose.

4. Fibres selon l'une quelconque des revendications 1 à 3, dans laquelle la carboxyméthylcellulose a un degré de carboxyméthylation d'au moins 0,65.

5. Fibres selon la revendication 1, dans laquelle l'alginate a une teneur en guluronate de 50-80% en poids et une teneur en M de 20-50% en poids.

6. Fibres selon l'une quelconque des revendications 1 à 5, dans laquelle la valeur de z est d'au moins 85%; optionnellement dans laquelle la valeur de z est d'au moins 90%.

7. Fibres selon l'une quelconque des revendications 1 à 6 produites par le co-filage d'une solution à filer aqueuse comprenant de la carboxyméthylcellulose dissoute et de l'alginate dissous dans un bain de coagulation.

8. Fibres selon l'une quelconque des revendications 1 à 7 sous une forme de brin.

9. Matériau non-tissé qui comprend des fibres entremêlées selon l'une quelconque des revendications précédentes.

10. Matériau non-tissé selon la revendication 9 sous la forme d'un feutre aiguilleté non-tissé; optionnellement en combinaison avec un substrat dans lequel les fibres du matériau sont aiguilletées.

11. Matériau tissé comprenant des fibres selon l'une quelconque des revendications 1 à 8.

12. Matériau tricoté comprenant des fibres selon l'une quelconque des revendications 1 à 8.

13. Pansement pour blessure comprenant un matériau selon l'une quelconque des revendications 9 à 12; optionnellement dans lequel ledit matériau forme une couche d'un pansement à couches multiples.

14. Procédé pour la production de fibres comprenant le filage d'une solution à filer qui est une solution aqueuse contenant de la carboxyméthylcellulose dissoute et de l'alginate dissous dans un bain de coagulation contenant des ions calcium dissous qui entraînent la réticulation de l'alginate et la précipitation de fibres composées de l'alginate et du polysaccharide **caractérisées en ce que**:

(a) la quantité de carboxyméthylcellulose présente dans la solution à filer est d'au moins 50% en poids du poids total de la carboxyméthylcellulose et de l'alginate,
(b) l'alginate de sodium a une teneur en guluronate de 50-90% en poids,
(c) la concentration d'ions calcium dans le bain de coagulation fournit des fibres dans lesquelles la valeur de z dans l'expression:

$$z = \frac{(2x/40)}{(2x/40)+(y/23)} \qquad (1)$$

où x et y sont les pourcentages en poids de calcium et de sodium respectivement dans les fibres, est d'au moins 80%, telle que déterminée par une spectroscopie d'absorption atomique, et
(d) les fibres produites contiennent la carboxyméthylcellulose et l'alginate dans une quantité totale d'au moins 90% en poids de la fibre.

15. Procédé selon la revendication 14, dans lequel la quantité de carboxyméthylcellulose dans la solution à filer est d'au moins 55% en poids du poids total de la carboxyméthylcellulose et de l'alginate; optionnellement dans lequel la quantité de carboxyméthylcellulose dans la solution à filer est d'au moins 60% en poids du poids total de la carboxyméthylcellulose et de l'alginate.

## 85% Alginate / 15% CMC   45% Alginate / 55% CMC

**Fig. 1**

EP 3 377 682 B1

**EP 3 377 682 B1**

**REFERENCES CITED IN THE DESCRIPTION**

**Patent documents cited in the description**

- EP 0616650 A
- US 6080420 A
- US 6140257 A